# EUROPEAN PATENT APPLICATION

(11) **EP 4 563 144 A1**
(43) Date of publication of application: **04.06.2025**
(21) Application number: 24216043.0
(22) Date of filing: 28.11.2024
(51) Int. Cl.: A61K 9/20, A61K 9/28, A61K 31/155

(54) **A TABLET COMPRISING AN EXTENDED-RELEASE FORMULATION FOR METFORMIN AND IMMEDIATE RELEASE FORMULATION COMPRISING SITAGLIPTIN**

(30) Priority: 30.11.2023 TR 202316131
(62) Divisional of application: 24222540.7
(71) Applicant: Sanovel Ilac Sanayi ve Ticaret A.S., Sisli - Istanbul (TR)
(72) Inventor: SUNEL, Fatih, Istanbul (TR); PEHLIVAN AKALIN, Nur, Istanbul (TR); TUNC, Guldeniz, Istanbul (TR)
(74) Representative: Genç Ilhan, Oznur

(57) **Abstract**

The present invention relates to a tablet formulation comprising a core tablet having metformin and at least one matrix agent and a coating solution having sitagliptin surrounding the core tablet and a film coating wherein the coating solution further comprises at least one antioxidant and at least one coating agent. Further, the present invention provides a method for the preparation of said composition.

## Description

### Field of the invention

The present invention relates to a tablet formulation comprising a core tablet having metformin and at least one matrix agent and a coating solution having sitagliptin surrounding the core tablet and a film coating wherein the coating solution further comprises at least one antioxidant and at least one coating agent. Further, the present invention provides a method for the preparation of said composition.

### Background of the invention

Diabetes mellitus is a group of disorders of carbohydrate metabolism in which the action of insulin is diminished or absent through altered secretion, decreased insulin activity or a combination of both factors. There are two main types of diabetes; Type 1 and Type 2:
Type 1 diabetes occurs because the insulin-producing cells of the pancreas (beta cells) are damaged. In Type 1 diabetes, the pancreas makes little or no insulin, so sugar cannot get into the body's cells for use as energy.
In Type 2 diabetes, the pancreas makes insulin, but it either doesn't produce enough, or the insulin does not work properly. Type 2 diabetes may sometimes be controlled with a combination of diet, weight management, and exercise. However, treatment also may include oral glucose-lowering medications or insulin injections.

Metformin is antidiabetics having an orally-administrated biguanide structure. Metformin hydrochloride is a white to off-white crystalline compound and it is freely soluble in water and practically insoluble in acetone, ether, and chloroform. The chemical name of metformin is 1,1-dimethyl biguanide, has the following chemical structure of Formula I.

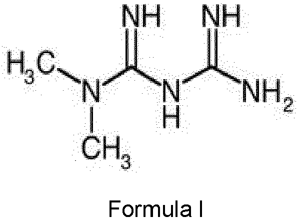

Although metformin is effective at lowering blood glucose levels, its use is associated with gastrointestinal (GI) adverse effects, particularly diarrhea and nausea. These adverse effects may limit the tolerated dose of metformin and cause patients to discontinue the therapy.

Sitagliptin is indicated as an adjunct to diet and exercise to improve glycemic control in adults with type 2 diabetes mellitus. It is an oral antihyperglycemic of the dipeptidyl peptidase-4 (DPP-4) inhibitor class. DPP-4 inhibitors work by blocking the action of DPP-4, an enzyme which destroys the hormone incretin. There are two types of incretin hormones found in the body, called glucagon-like peptide-1 (GLP-1) and glucose-dependent insulinotropic peptide (GIP). These hormones are naturally produced by the body in response to food intake. Their function is to help the body produce more insulin only when it is needed and reduce the amount of glucose being produced by the liver when it is not needed. Sitagliptin works by binding to DPP-4 and preventing it from breaking down the GLP-1 and GIP. This increases the levels of these hormones in the body and so increases their effect on controlling blood sugar.

The chemical name of sitagliptin is (3R)-3-amino-1-[3-(trifluoromethyl)-6,8-dihydro-5H-[1,2,4]triazolo[4,3-a]pyrazin-7-yl]-4-(2,4,5-trifluorophenyl) butan-1-one or (2R)-4-oxo-4- [3-(trifluoromethyl)-5,6-dihydro[1,2,4]triazolo[4,3-a]pyrazin-7(8H)-yl]-1-(2,4,5trifluorophenyl) butan-2-amine) and its chemical structure is shown in the Formula II.

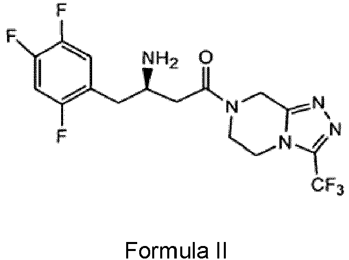

Sitagliptin is disclosed in the U.S. Patent No. 6699871. A crystal phosphate monohydrate form of sitagliptin is disclosed in the patent WO 2005003135.

DPP-4 inhibitors, especially sitagliptin have a novel mechanism of action and many studies showing their efficacy and safety in medication are available.

Sitagliptin increases plasma GLP-1 concentration and elevates cellular cAMP levels in pancreatic beta-cells leading to potentiate insulin secretion, whereas metformin improves glucose tolerance in patients with Type 2 diabetes, lowering both basal and postprandial plasma glucose. Its pharmacologic mechanisms of action are different from other classes of oral antihyperglycemic agents in that metformin decreases hepatic glucose production, decreases intestinal absorption of glucose and improves insulin sensitivity by increasing peripheral glucose uptake and utilization. Therefore, they both help to reduce blood glucose levels in different pathways in type 2 diabetes patients.

Extended-release formulations of metformin have advantages over immediate release in terms of affording a more uniform maintenance of blood plasma active drug concentrations and providing better patient compliance by reducing the frequency of administration required.

A tablet formulation for sitagliptin and extended-release metformin combination is commercially available under the trade name Janumet XR^{®}.

PCT publication WO 2009111200 discloses the tablet comprising the combinations of an extended-release form of metformin, or a pharmaceutically acceptable salt thereof, coated with an immediate-release form of sitagliptin, or a pharmaceutically acceptable salt thereof.

WO 2009099734 discloses pharmaceutical compositions providing an extended release of metformin and an immediate release of sitagliptin. The tablet core is comprised of metformin and an extended release polymer (HPMC). The tablet core is then coated with immediate release polymer comprising sitagliptin.

There is also still a need for a tablet form comprising an extended-release formulation for metformin and immediate release formulation for sitagliptin having desired dissolution profile and stability.

In the present invention, the dosage form of pharmaceutical combination is a core tablet coating with a solution comprising the active ingredient to overcome the problem of incompatibility and dissolution rate with a formulation appropriate to the characteristics of the active ingredients.

### Detailed description of the Invention

The main object of the present invention is to avoid incompatibility problems between metformin and sitagliptin, and adding additional advantages to the prior art.

It is a further object of the present invention to provide a tablet formulation comprising delivery of a first active drug as an extended-release formulation for metformin in combination with delivery of a second active drug by immediate release comprising sitagliptin which having the desired dissolution profile and stability.

Another object of the present invention is to provide a process for preparing a tablet comprising metformin and sitagliptin. The process is a simple, rapid, cost effective, timesaving, and industrially convenient method.

As used herein, the term "metformin" includes metformin and pharmaceutically acceptable salts thereof. The pharmaceutically acceptable salts comprise hydrochloride, hydrobromide, fumarate, malonate, malate, succinate, lactate, glycolate, maleate, citrate, and aspartate. The pharmaceutically acceptable salts of metformin may be present in hydrous or anhydrous forms. They may also be present in crystalline or amorphous forms. In this invention, preferably metformin hydrochloride is used.

As used herein, the term "sitagliptin" includes sitagliptin and pharmaceutically acceptable salts thereof. The pharmaceutically acceptable salts comprise hydrochloride, hydrobromide, phosphate, sulphate, mesylate, besylate, tosylate, fumarate, malonate, malate, succinate, lactate, glycolate, maleate, citrate, and aspartate. The pharmaceutically acceptable salts of sitagliptin may be present in hydrous or anhydrous forms. The hydrate forms may be monohydrate or dihydrate forms. The pharmaceutically acceptable salts of sitagliptin may also be present in crystalline or amorphous forms. In this invention, preferably sitagliptin phosphate monohydrate is used.

The term "core" as used herein refers to a compact mass having a definite geometric shape such as tablets, granules, pellets, capsules.

When metformin and sitagliptin are taken at the same time, there might be inconveniences due to the differences in the half-life duration of active agents. Thus, the pharmaceutical combination comprises metformin and sitagliptin wherein the pharmaceutical composition has compartments comprising active agents with different release profiles.

Sitagliptin and metformin combination has incompatibility and stability problems due to interactions between active agents. Separating metformin and sitagliptin in such a way that their interaction is eliminated is an effective solution so as to overcome these problems. For this purpose, we compressed a mixture comprising metformin as a core tablet, then we coated it with a solution comprising sitagliptin.

In the invention, when sitagliptin or a pharmaceutically acceptable salts thereof and at least one coating agent and at least one antioxidant is dissolved in a coating solvent, a solution comprising a higher proportion and homogenity of sitagliptin or a pharmaceutically acceptable salts thereof is obtained, this minimizes active substance losses during granulation and provides more homogeneous (content uniformity) and efficient tablet formulation.

Also, as known, metformin is a very poorly compressible active substance and in the present invention, metformin presents in high amounts in the core tablet. It was surprisingly found that when prepared the core tablet with a coating solution comprising sitagliptin or a pharmaceutically acceptable salts thereof, it was observed that the desired tablet hardness and compressibility of metformin HCl is provided. So, an easy method was created to eliminate the disadvantages of both active ingredients. The provides the desired stability.

According to one embodiment of the present invention, a tablet formulation comprises;
- A core tablet comprising metformin and at least one matrix agent,
- A coating solution comprising sitagliptin surrounding the core tablet,
- A film coating

Wherein the coating solution further comprises at least one antioxidant and at least one coating agent.

According to one embodiment of the present invention, metformin is present metformin hydrochloride form in the tablet, sitagliptin is present sitagliptin phosphate monohydrate in the tablet.

According to one embodiment of the present invention, the compartments are in direct contact with each other. In this invention, the compartments are in the form of core or coating. The first compartment is a tablet core which comprises metformin. The second compartment is sitagliptin solution coating, the third compartment is the film coating.

According to one embodiment of the present invention, the amount of metformin is between 50.0% and 75.0% by weight of the total tablet. The amount of sitagliptin is between 3.0% and 15.0% by weight of the total tablet.

According to one embodiment of the present invention, preferably, the amount of metformin is between 55.0% and 63.0% by weight of the total tablet and the amount of sitagliptin is between 6.0% and 9.5% by weight of the total tablet.

Said matrix agent is selected from the group comprising hydroxypropyl methyl cellulose, hydroxyethyl cellulose, hydroxypropyl cellulose, methyl cellulose, galactomannanes, guar gum, carob gum, gum arabicum, alginates, pectins, polyvinylacetate, acrylic acid polymers, polyethylene oxide, white wax, bees wax, carnauba wax, stearic acid, palmitic acid, lauric acid, cetyl alcohol, cetostearyl alcohol, stearyl alcohol, ethyl cellulose, acrylic acid polymers and copolymers (available commercially under Eudragit<^{®}>brand) or mixtures thereof.

According to one embodiment of the present invention, the matrix agent is hydroxypropyl methyl cellulose K100 MCR/ K100M/ CN10T. The flowability and compressibility of metformin is poor. In the present invention, granulating metformin with the help of selected matrix agent provides desired flowability and compressibility of metformin.

According to one embodiment of the present invention, the amount of matrix agent is between 8.0% and 25.0%, preferably it is between 12.0% and 20.0% by weight of the total tablet.

Suitable antioxidants are selected from the group comprising propyl gallate, alpha tocopherol, ascorbic acid, ascorbyl palmitate, butylhydroxyanisole (BHA), butylhydroxytoluene (BHT), erythorbic acid, monothioglycerol, potassium metabisulfite, sodium ascorbate, sodium metabisulfite, sodium sulfite, thymol or mixtures thereof.

According to one embodiment of the present invention, used antioxidant in the coating solution comprising sitagliptin is propyl gallate.

According to one embodiment of the present invention, used the coating agents in the coating solution comprising sitagliptin are polyethylene glycol, hydroxypropyl methyl cellulose, kaolin or mixture thereof.

According to one embodiment of the present invention, the core further comprises at least one binder.

Suitable binders are selected from group comprising carboxymethyl cellulose, hydroxypropyl methyl cellulose, polyvinylpyrrolidone, microcrystalline cellulose, lactose monohydrate, starch, dibasic calcium phosphate, tribasic calcium phosphate, sodium carbonate, sodium bicarbonate, calcium carbonate, polydextrose, polyethylene oxide, hydroxypropyl methyl cellulose, methyl cellulose, polyethylene glycol or mixtures thereof.

According to one embodiment of the present invention, the amount of binders is between 1.0% and 15.0%, preferably it is between 1.0% and 8.0%, more preferably it is between 2.0% and 6.0% by weight of the total tablet.

According to one embodiment of the present invention, the binder is carboxymethyl cellulose or hydroxypropyl methyl cellulose (K100 LV) or mixtures thereof.

According to one embodiment of the present invention, the core further comprises at least one lubricant.

Suitable lubricants are selected from group comprising magnesium stearate, hydrophobic silicon dioxide, talc, sodium stearyl fumarate, polyethylene glycol, sodium lauryl sulphate, magnesium lauryl sulphate, fumaric acid, glyceryl palmitostearate, hydrogenated natural oils, zinc stearate, calcium stearate, silica, stearic acid, paraffin or mixtures thereof.

According to one embodiment of the present invention, the amount of lubricants is between 0.1% and 4.0% by weight, preferably 0.1% and 2.0% by weight of the total tablet.

According to one embodiment of the present invention, the lubricant is sodium stearyl fumarate.

Suitable coating agents are selected from the group comprising polymethacrylates, polydextrose, polyalkylacrylates copolymers, triacetin, hydroxylpropyl methyl cellulose, medium chain triglycerides, kaolin, hydroxypropyl cellulose, carnauba wax, polyvinyl alcohol, polyethylene glycol, talc, polyvinyl alcohol-polyethylene glycol copolymers (Kollicoat^{®} IR), ethylcellulose dispersions (Surelease^{®}), polyvinylprolidone, polyvinylprolidone-vinyl acetate copolymer (PVP-VA), all kinds of Opadry^{®}, pigments, dyes, titanium dioxide, red iron oxide, black iron oxide or mixtures thereof.

According to one embodiment of the present invention, the film coating is prepared with coating agents. Used the coating agents in the film coating are polyethylene glycol, hydroxypropyl cellulose, titanium dioxide, indigo dye, carnauba wax, or mixture thereof.

According to one embodiment of the present invention, the ratio of the core tablet to the coating solution comprising sitagliptin is in the range of between 1 and 10, preferably between 2.5 and 8.0 by weight. This ratio provides desired dissolution profile of active agents and desired combination efficacy.

According to this embodiment of the invention, the core tablet is between 75.0% and 85.0%, preferably between 77.0% and 82.0% by weight of the total tablet.

According to this embodiment of the invention, the coating solution comprising sitagliptin is between 12.0% and 24.0%, preferably between 15.0% and 20.0% by weight of the total tablet.

According to this embodiment of the invention, the film coating is between 1.5% and 4.0% by weight of the total tablet.

According to one embodiment of the present invention, sitagliptin and metformin combination are stable. The combination does not show incompatibilities, degradation problems, or extraction problems.

According to one embodiment of the present invention, a tablet formulation comprises;
- A core tablet comprising metformin HCl having carboxymethylcellulose sodium and hydroxypropyl methylcellulose (K100 LV) as binders, hydroxypropyl methylcellulose K100 MCR/ K100M/ CN10T as matrix agent, sodium stearyl fumarate as lubricant,
- A coating solution comprising sitagliptin phosphate monohydrate surrounding the core tablet having propyl gallate as antioxidant and coating agents,
- A film coating.

According to one embodiment of the present invention, a tablet formulation comprises;
- A core tablet comprising metformin HCl having hydroxypropyl methylcellulose K100 MCR/ K100M/ CN10T as matrix agent, sodium stearyl fumarate as lubricant,
- A coating solution comprising sitagliptin phosphate monohydrate surrounding the core tablet having propyl gallate as antioxidant and coating agents,
- A film coating.

According to another embodiment of the present invention, there is provided a process for preparation of the tablet formulation comprising metformin and sitagliptin, wherein the tablet is prepared by a process comprising the steps of:
a) preparing a core comprising metformin HCl which is obtained wet granulation or direct compression,
b) applying a coating solution comprising sitagliptin phosphate monohydrate surrounding the core which is obtained wet granulation,
c) applying a film coating.

### Example 1

| | | **(%) amount (w/w)** | **(%) amount (w/w)** |
|---|---|---|---|
| **Core** | | **75.0 - 85.0** | **80.3** |
| Metformin HCl | | 50.0 - 75.0 | 59.5 |
| Carboxymethylcellulose sodium (7 HF) | | 1.0 - 8.0 | 3.0 |
| Hydroxypropyl methylcellulose (K100 LV) | | 0.5 - 6.0 | 1.2 |
| Hydroxypropyl methylcellulose K100 MCR/ K100M/ CN10T | | 10.0 - 25.0 | 16.3 |
| Sodium stearyl fumarate | | 0.1 - 4.0 | 0.4 |
| **Sitagliptin solution coating** | | **12.0 - 24.0** | **17.3** |
| Sitagliptin | | 3.0 - 15.0 | 7.6 |
| Propyl gallate | | 0.01 - 2.0 | 0.1 |
| Opadry Clear | | 4.0 - 15.0 | 9.5 |
| | -Polyethylene glycol | | |
| | -Kaolin | | |
| | - Hydroxypropyl methyl cellulose | | |
| **Film coating** | | **1.5 - 4.0** | **2.3** |
| Hydroxypropyl Cellulose | | | |
| Macrogol 3350 | | | |
| Titanium Dioxide | | | |
| Carnauba wax | | | |
| Indigo Dye | | | |
| | **Total Tablet** | **100** | **100** |

A process for preparing the tablet in example 1 comprises the following steps:

### Core tablet

a) Mixing Metformin HCl, carboxymethylcellulose sodium and hydroxypropyl methylcellulose (K100 LV),
b) Adding pure water and granulating,
c) Drying the granules, then sieving,
d) Adding hydroxypropyl methylcellulose K100 MCR/ K100M/ CN10T into the mixture and mixing,
e) Then, adding sodium stearyl fumarate and mixing,
f) Compressing the mixture to form a core.

### Sitagliptin solution coating

g) Preparing a coating solution with a coating material (preferably, Opadry Clear comprising polyethylene glycol and kaolin and hydroxypropyl methyl cellulose),
h) Adding propyl gallate into the coating solution and mixing,
i) Adding sitagliptin and then obtained the coating solution comprising sitagliptin,
j) Coating the core tablet with the solution coating comprising sitagliptin.

### Film coating

k) preparing a film coating with hydroxypropyl cellulose, macrogol 3350, titanium dioxide, carnauba wax, indigo dye,
l) Coating at step (j) coated tablet with the film coating.

### Example 2

| | | **(%) amount (w/w)** | **(%) amount (w/w)** |
|---|---|---|---|
| **Core** | | **75.0 - 85.0** | **81.7** |
| Metformin HCl DC %95 | | 50.0 - 75.0 | 64.50 |
| Hydroxypropyl methylcellulose K100 MCR/ K100M/ CN10T | | 10.0 - 25.0 | 16.8 |
| Sodium stearyl fumarate | | 0.1 - 4.0 | 0.4 |
| **Sitagliptin solution coating** | | **12.0 - 24.0** | **15.88** |
| Sitagliptin | | 3.0 - 15.0 | 7.87 |
| Propyl gallate | | 0.01 - 2.0 | 0.15 |
| Opadry Clear | | 4.0 - 15.0 | 7.86 |
| | -Polyethylene glycol | | |
| | -Kaolin | | |
| | - Hydroxypropyl methyl cellulose | | |
| **Film coating** | | **1.5 - 4.0** | **2.42** |
| Hydroxypropyl Cellulose | | | |
| Macrogol 3350 | | | |
| Titanium Dioxide | | | |
| Carnauba wax | | | |
| Indigo Dye | | | |
| | **Total Tablet** | **100** | **100** |

A process for preparing the tablet in example 2 comprises the following steps:

### Core tablet

a) Sieving Metformin HCl DC %95 into 1.5 mm,
b) Adding hydroxypropyl methylcellulose K100 MCR/ K100M/ CN10T into the mixture and mixing,
c) Then, adding sodium stearyl fumarate and mixing,
d) Compressing the mixture to form a core.

### Sitagliptin solution coating

e) Preparing a coating solution with a coating material (preferably, Opadry Clear comprising polyethylene glycol and kaolin and hydroxypropyl methyl cellulose) and pure water,
f) Dissolving propyl gallate into ethanol and adding the mixture into the coating solution and mixing,
g) Adding sitagliptin and then obtained the coating solution comprising sitagliptin,
h) Coating the core tablet with the solution coating comprising sitagliptin.

### Film coating

i) preparing a film coating with hydroxypropyl cellulose, macrogol 3350, titanium dioxide, carnauba wax, indigo dye,
j) Coating at step (j) coated tablet with the film coating.

## Claims

1. A tablet formulation comprising;
- A core tablet comprising metformin and at least one matrix agent,
- A coating solution comprising sitagliptin surrounding the core tablet,
- A film coating
wherein the coating solution further comprises at least one antioxidant and at least one coating agent.

2. The tablet formulation according to claim 1, wherein metformin is present metformin hydrochloride form in the tablet, sitagliptin is present sitagliptin phosphate monohydrate in the tablet.

3. The tablet formulation according to claim 1, wherein the amount of metformin is between 50.0% and 75.0% by weight of the total tablet and the amount of sitagliptin is between 3.0% and 15.0% by weight of the total tablet.

4. The tablet formulation according to claim 1, wherein matrix agent is selected from the group comprising hydroxypropyl methyl cellulose, hydroxyethyl cellulose, hydroxypropyl cellulose, methyl cellulose, galactomannanes, guar gum, carob gum, gum arabicum, alginates, pectins, polyvinylacetate, acrylic acid polymers, polyethylene oxide, white wax, bees wax, carnauba wax, stearic acid, palmitic acid, lauric acid, cetyl alcohol, cetostearyl alcohol, stearyl alcohol, ethyl cellulose, acrylic acid polymers and copolymers (available commercially under Eudragit<^{®}>brand) or mixtures thereof.

5. The tablet formulation according to claim 1 or 4, wherein the matrix agent is hydroxypropyl methyl cellulose K100 MCR/ K100M/ CN10T.

6. The tablet formulation according to claim 4 or 5, wherein the amount of matrix agent is between 8.0% and 25.0%, preferably it is between 12.0% and 20.0% by weight of the total tablet.

7. The tablet formulation according to claim 1, wherein antioxidants are selected from the group comprising propyl gallate, alpha tocopherol, ascorbic acid, ascorbyl palmitate, butylhydroxyanisole (BHA), butylhydroxytoluene (BHT), erythorbic acid, monothioglycerol, potassium metabisulfite, sodium ascorbate, sodium metabisulfite, sodium sulfite, thymol or mixtures thereof.

8. The tablet formulation according to claim 7, wherein used antioxidant in the coating solution comprising sitagliptin is propyl gallate.

9. The tablet formulation according to claim 1, wherein, used the coating agents in the coating solution comprising sitagliptin are polyethylene glycol, hydroxypropyl methyl cellulose, kaolin or mixture thereof.

10. The tablet formulation according to claim 1, wherein the core further comprises at least one binder.

11. The tablet formulation according to claim 10, wherein binders are selected from group comprising carboxymethyl cellulose, hydroxypropyl methyl cellulose, polyvinylpyrrolidone, microcrystalline cellulose, lactose monohydrate, starch, dibasic calcium phosphate, tribasic calcium phosphate, sodium carbonate, sodium bicarbonate, calcium carbonate, polydextrose, polyethylene oxide, hydroxypropyl methyl cellulose, methyl cellulose, polyethylene glycol or mixtures thereof.

12. The tablet formulation according to claim 11, wherein the binder is carboxymethyl cellulose or hydroxypropyl methyl cellulose (K100 LV) or mixtures thereof.

13. The tablet formulation according to claim 1, wherein the core further comprises at least one lubricant.

14. The tablet formulation according to claim 1, the ratio of the core tablet to the coating solution comprising sitagliptin is in the range of between 1 and 10, preferably between 2.5 and 8.0 by weight.

15. The tablet formulation according to claim 1, wherein a tablet formulation comprising;
- A core tablet comprising metformin HCl having hydroxypropyl methylcellulose K100 MCR/ K100M/ CN10T as matrix agent, sodium stearyl fumarate as lubricant,
- A coating solution comprising sitagliptin phosphate monohydrate surrounding the core tablet having propyl gallate as antioxidant and coating agents,
- A film coating.
